# EUROPEAN PATENT APPLICATION

(11) **EP 1 614 402 A1**
(43) Date of publication of application: **11.01.2006**
(21) Application number: 04254131.8
(22) Date of filing: 09.07.2004
(51) Int. Cl.: A61F 2/44, A61F 2/02

(54) **Patch material for inervertebral disc annulus defect repair**

(71) Applicant: Tsou, Paul M., Santa Monica, California 90402 (US)
(72) Inventor: Tsou, Paul M., Santa Monica, California 90402 (US)
(74) Representative: Roberts, Gwilym Vaughan

(57) **Abstract**

A patch (10, 100) for repairing defects in a intervertebral disc annulus, including a substantially thin layer (11, 25) of animal collagen arranged in a substantially circular shape, a fabric layer (12) disposed beneath the layer of animal collagen and coupled to the layer of animal collagen by biological glue, a barrier layer (13, 26) of liquid resistant material disposed beneath the fabric layer, and coupled to the fabric layer by an adhesive, a plurality of flexible fibers disposed on a top surface of the patch and oriented in a radial direction and at least one suture (82) disposed through the patch, the suture acting as a handle.

## Description

### BACKGROUND OF THE INVENTION

### 1. Field of the Invention

The present invention relates to the field of spinal surgery, and more particularly to a patch (and patch material) which can be used to repair defects (natural, traumatic or surgical) in a intervertebral disc annulus.

### 2. Prior Art

Nucleus pulposus is the jelly-like substance located in the center of the human intervertebral disc. The other components of the disc are the end-plates and the annulus fibrosus. The nucleus is sealed off from nerve endings, segmental spinal nerve elements and vascular tree by the multi-layered annulus fibrosus in its periphery, and by the cartilaginous-osseous end-plates at its cranial and caudad ends. The metabolic turn over of nucleus pulposus is by a slow diffusion process from its nearest capillaries in the outer surfaces of the end-plates, and to a lesser extend through the annulus. The bulk dried weight of the nucleus is made-up of proteoglycan, a hydrophilic complex protein molecule. Proteoglycan and its catabolic byproducts are extremely inflammatory to the nerve elements and vascular membranes. Substantial leakage of proteoglycan from its usual containment structures will cause intense inflammatory reaction of the nerve elements and blood vessels. Inflammation of these structures leads to back pain and sciatica-like leg pain symptoms.

Breach of annulus or endplate barrier integrity may occur as a natural degenerative process, traumatic event, surgical intervention or a combination of the above. Minimal access visualized intradiscal surgery technique for the treatment of herniated disc and chronic discogenic pain requires a surgically created annular opening into the disc space. Using this technique, some postoperative extremity dysesthesia can be caused by nerve inflammation secondary to proteoglycan leakage from the surgically created annulotomy hole. Surgically created holes in the annulus fibrosus are also made in various other surgical procedures to lumbar and cervical discs.

It is therefore an object of this invention to provide a method and device for the repair of structural openings and defects in the annulus fibrosus of the lumbar, cervical and thoracic spine. Such defects can be naturally-occurring or surgically created. One method of minimally-invasive spinal surgery which creates openings in the annulus fibrosis which can be repaired by the patch of the present invention is disclosed in United States patent application serial number 09/997,361 entitled "Method and Apparatus for Transforaminal Endoscopic Surgery," filed November 30, 2001.

It is another object of this invention to provide a patch material which is easy to use and can be inserted into the disc without causing additional damage to the annulus fibrosis.

It is yet another object of this invention to provide a patch and patch material which includes naturally-occurring substances, and can be placed in the human body with little chance of rejection.

The present invention is as claimed in the independent claims. Preferred or optional features are as stated in the dependent claims.

In an embodiment, the patch may be used to seal defects in the annulus fibrosis. The patch may be substantially thin and flexible, and may be manufactured from animal collagen materials. The patch may be substantially circular in shape, although other shapes may be used, depending on the size and shape of the opening to be sealed. The patch may also include a layer of fabric, and a layer of barrier material. The patch may be provided with thin, flexible, filaments on its upper surface so that it remains flat after it is inserted into place.

Various aspects of the invention will now be described by way of non-limiting examples, and with reference to the following drawings.

### BRIEF DESCRIPTION OF THE DRAWINGS

Figure 1 illustrates a patch for repairing defects in an intervertebral disc annulus according to the present invention.
Figure 2 illustrates a surgical approach to locating an annular insertion point for transforaminal endoscopic lumbar surgery.
Figure 3 is a cross-sectional view of a disc showing fissures in a damaged or degenerative annulus fibrosus.
Figure 4 is a cross-sectional view of a disc showing a disc herniation and a method of surgical extraction.
Figure 5 is a cross-sectional view of a disc showing the use of a laser to seal fissures in a defective annulus fibrosus.
Figure 6 is a cross-sectional view of a disc showing the use of a radio-frequency probe to seal fissures in a damaged annulus fibrosus.
Figure 7a illustrates a round annulotomy in a disc annulus.
Figure 7b illustrates a multilateral annulotomy in a disc annulus.
Figure 8 is a cross-sectional view of typical patch material.
Figure 9 illustrates a representative structure of patch for use in sealing a round annulotomy.
Figure 10 illustrates a representative structure of patch for use in sealing a multilateral annulotomy.

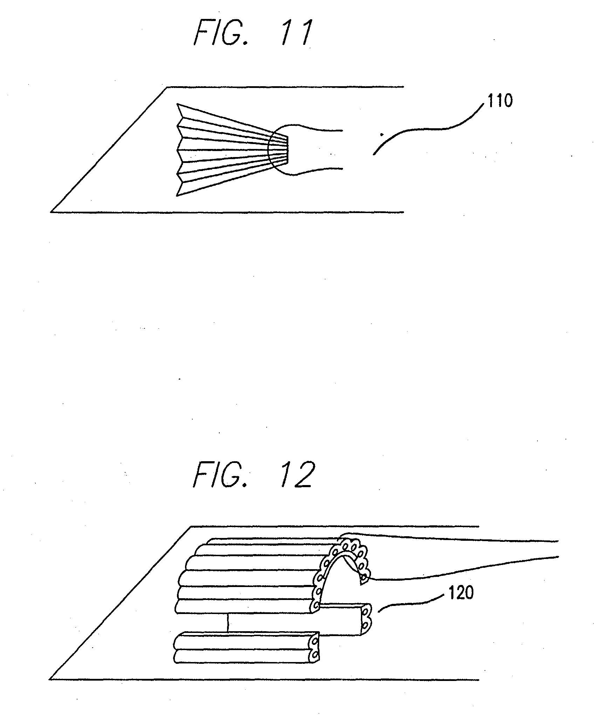

Figure 11 illustrates a method of inserting the patch illustrated in Figure 9.
Figure 12 illustrates a method of inserting the patch illustrated in Figure 10.
Figure 13 illustrates an alternative embodiment of the invention wherein the layers of fabric and biological material have been switched.
Figure 14 illustrates another alternative embodiment of the invention which includes only two layers in the patch.

### DETAILED DESCRIPTION OF THE PREFERRED EMBODIMENT

A novel patch and patch material which can be used to repair defects in an intervertebral disc annulus will be described. In the following description, various structural details, manufacturing methods and materials will be set forth in order to give a more thorough understanding of the present invention. It will be apparent to those of skill in the art that the present invention may be practiced without these specific details. In other instances, well known processes and structures are not specifically described so as not to obscure the present invention unnecessarily.

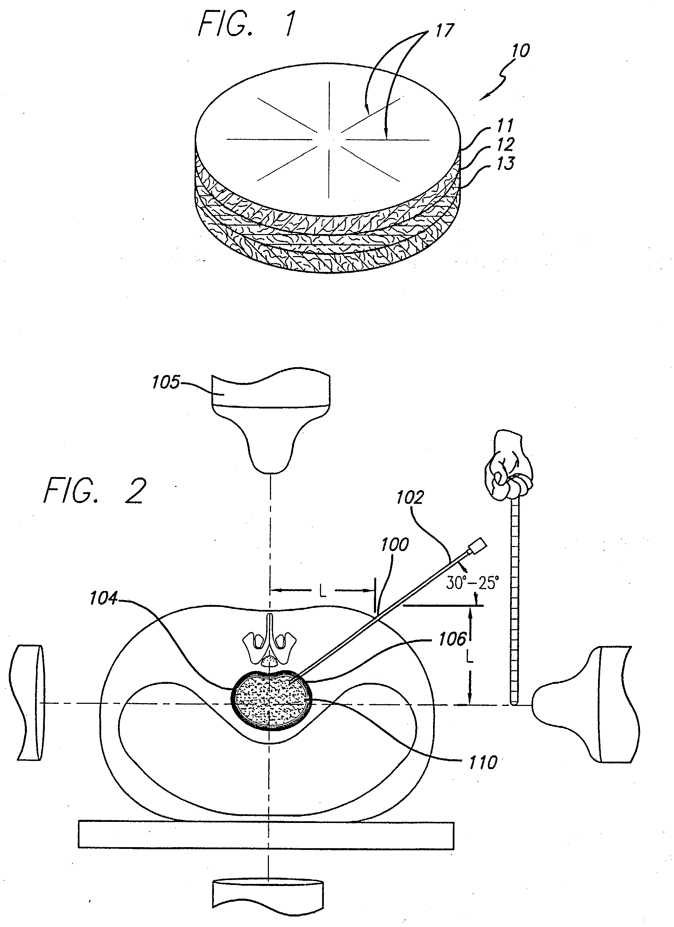

A perspective view of the preferred embodiment of the present invention is shown in Figure 1. Although the structure of the patch 10 will be described in detail below, it is helpful to understand the invention in the context of a surgical procedure used for the repair of intervertebral discs. An understanding of the surgical process will be helpful in understanding the use and operation of the patch of the present invention. It will be recognized that the patch 10 of the present invention is not limited to only those situations where an annulus defect is caused by surgery. The patch 10 can be used with equal effectiveness to repair defects that are natural (such as congenital defects) or are caused by trauma. Moreover, the present invention is not limited to the specific surgical procedure described. The patch 10 can be used in connection with almost any type of surgical procedure which results in a breach or defect in the annulus fibrosus.

The surgical method used in connection with the present invention is shown in Figure 2. There, the representative surgical operation is carried out using local anesthesia and conscious sedation. The skin window 100, or the apparatus point of entry, is first calculated. The window may be visualized on an x-ray image. From the skin window a needle 102 is inserted and guided to a location adjacent to the disc 104 to be repaired. This location is known as the annular window 106. In the preferred embodiment, an 18-gauge needle is inserted at an approximate 25-35 degree toward the annular window 106. The use of a computer assisted image guidance system 105 for the needle 102 is optional. In a successful approach, the needle 102 enters the disc 104 through the foraminal annular window 106. In a sequential exchange the needle is replaced by a thin guide pin, blunt obturator, beveled cannula, and the operating endoscope (not shown in Figure 2). Each instrument has a larger diameter than the predecessor. The cannula will generally have the largest diameter. In a typical surgery, the cannula will have diameter of approximately 7 millimeters. During the surgical process, an opening (or defect) will be created in the annulus fibrosus 110. This opening must be repaired, or the patient will suffer complication and pain that may be debilitating and severe.

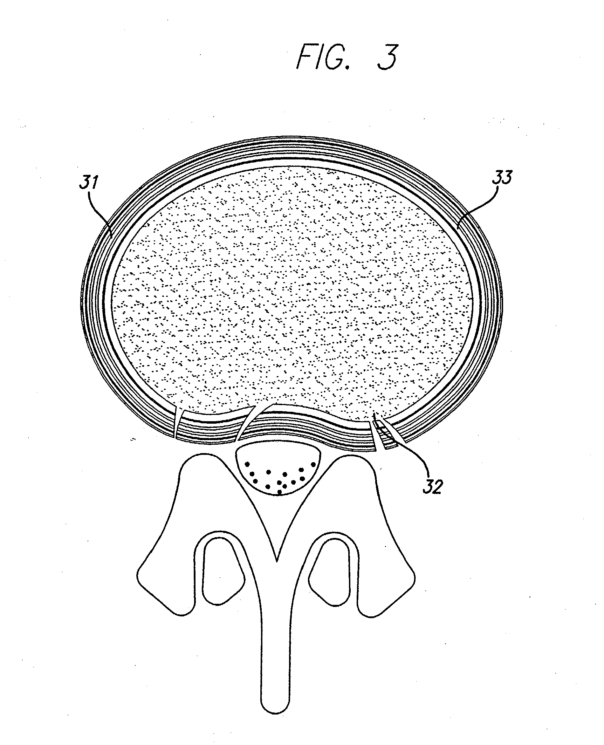

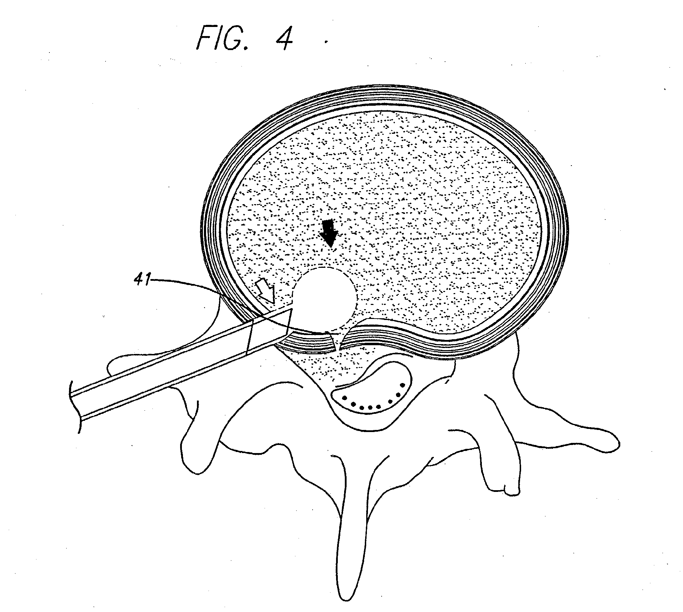

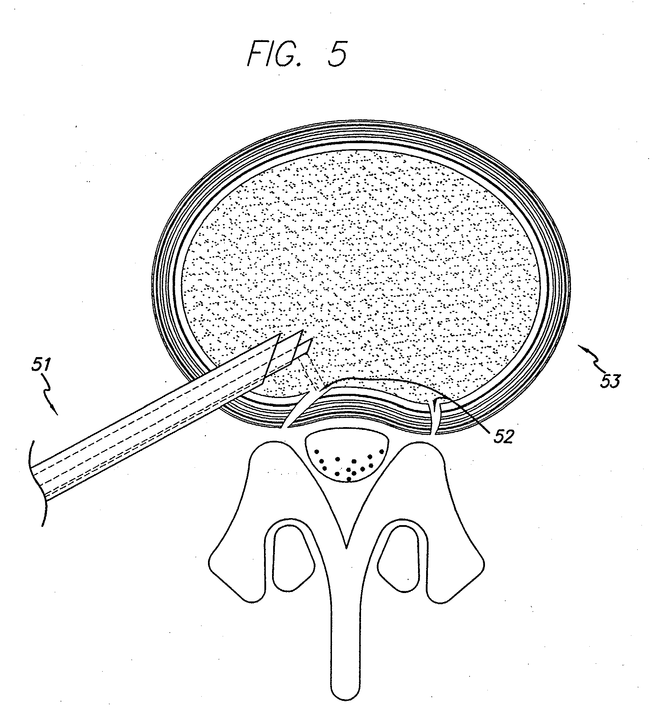

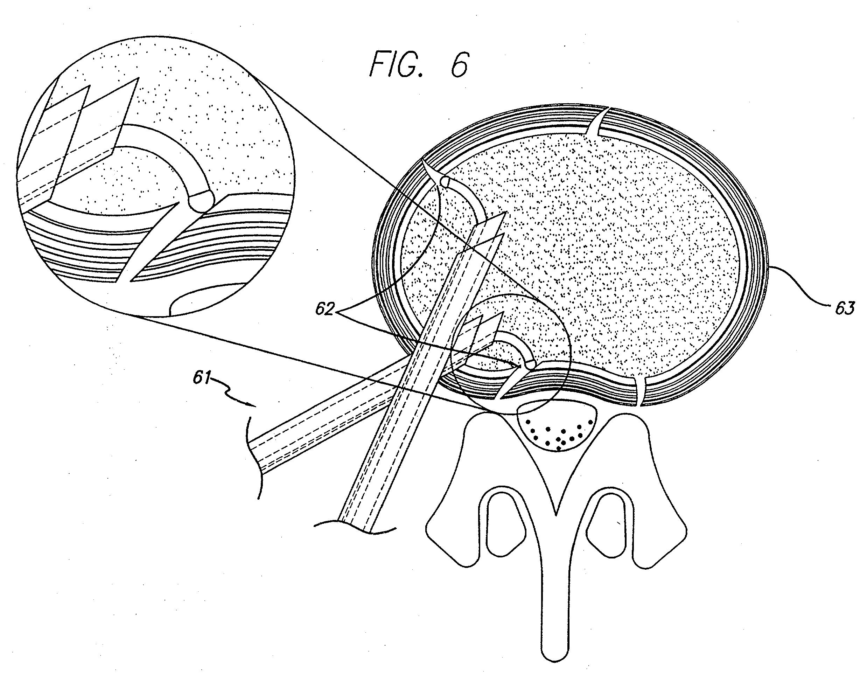

Figures 3 through 6 illustrate various different types of defects to the annulus pulposus that can be repaired with the use of the patch of the present invention. Figure 3 is a cross-sectional view of a disc 31 showing fissures 32 in a damaged or degenerative annulus fibrosus 33. Figure 4 shows a disc herniation 41 and a method of surgical extraction. Figure 5 illustrates the use of a laser 51 to seal fissures 52 in a defective annulus fibrosus 53. Finally, Figure 6 is a cross-sectional view of a disc showing the use of a radio-frequency probe 61 to seal fissures 62 in a damaged annulus fibrosus 63. It is to be understood that these types of defects are not the only ones that can be repaired with the use of the patch 10 of the present invention, but are presented only as examples of typical applications.

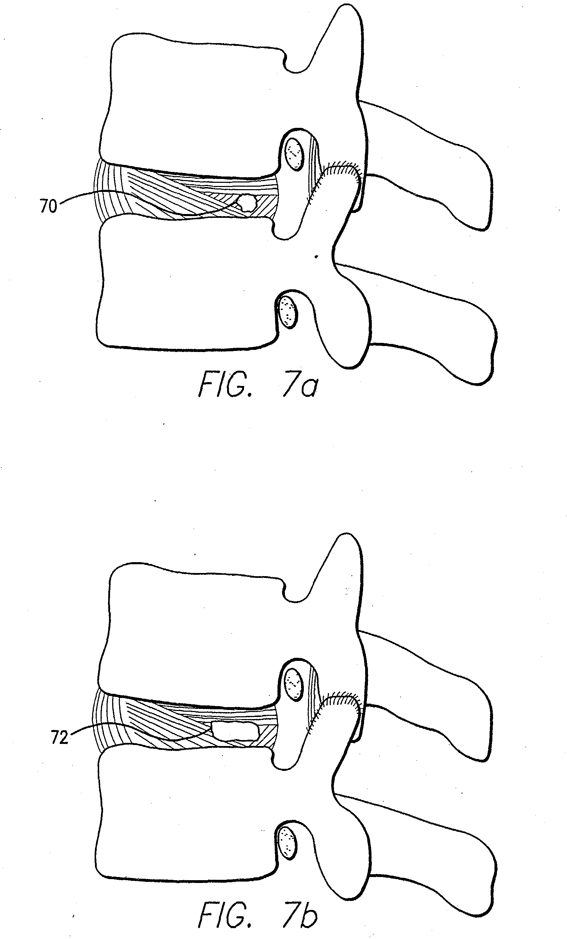

Referring again to Figure 1, an intervertebral disc patch 10 according to the present invention is illustrated. In the preferred embodiment, the patch 10 is substantially circular. A circular patch is best used to seal round annulotomy. A round annulotomy 71 is shown in Figure 7a. This is the type of defect that is typically formed in the annulus during surgical procedures such as the one described with reference to Figure 2. It is anticipated that in the large majority of instances, the patch 10 will be used in surgical procedures. Thus, the circular shape is preferred. Other shapes can also be used, as will be described in more detail below. The preferred embodiment of the patch includes three layers 11, 12 and 13. Each of the layers is a different material, and performs a different function.

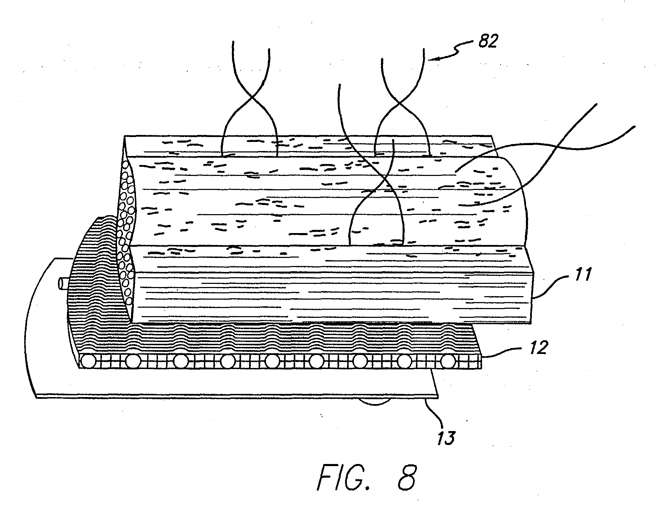

The characteristics of the patch 10 and its constituent material will now be described. A typical cross-sectional view of the patch is shown in Figure 8. As noted, the patch includes three layers 11,12, and 13. The major component of the patch is the top layer 11 which is made up of biological material. Biological material is the preferred material for the top layer because it acts as a repair scaffold by native fibroblasts and is ultimately replaced by native collagen. In the preferred embodiment, the top layer 11 of the patch 10 is manufactured from animal collagen fibers. The animal sources are easily available in domesticated animals such as cows, pigs and sheep. The anatomical location of the collagen fibers in the animals can be from the tendons, dura, intestine submucosa, and the like. In an alternative embodiment, the biological material consists of human collagen, either from the patient's own body, or from donor tissue.

Immediately under the layer of biological material 11 is a layer 12 that, in the preferred embodiment, is composed of fabric. The fabric provides a stable base to anchor the biological material. Fibers in the fabric hold the biological material in place, and give it the desired shape. The layers 11 and 12 are held together in the preferred embodiment by biological glue. Other adhesives (including synthetic adhesives) or glues can be used with equal effectiveness. Any number of different fabrics can be used for the middle layer 12. Nylon has been found to be particularly effective. Other types of materials which can be used include cotton and synthetic polymers such as rayon. Tightly woven fabrics are preferred.

Disposed under the middle layer 12 is the bottom layer 13 of the patch 10. The bottom layer 13 is a barrier material - such as silicone, plastic or the like - which is resistant to penetration by liquids. It has been found that plastic is most advantageously used to block the transfer (or leakage) of the nucleus pulposus through the patch 10. The barrier material in the bottom layer 13 works to keep all liquids within the disc structure, and not allow moisture to escape. The barrier layer 13 is also coupled to the middle fabric layer 12 by an adhesive or glue.

When the patch is in place over the defect, it initially acts as a physical barrier in preventing the escape of nucleus pulposus and its metabolites from within the disc. The collagen fibers in layer 11 also act as a scaffold for host fibroblast to migrate into the graft collage lattice, and then lay down new collagen fibers. Other types of host scavenger cells, including macrophages, will also migrate into the lattice and gradually remove the graft collagen. The sources of fiberblasts and macrophages are from outside of the disc via blood vessels. The original patch collagen ultimately is completely replaced by new host collagen. This is accomplished within a clinically reasonable amount of time.

It will be apparent to those of skill in the art that other materials can be used to manufacture the patch 10. For example, human allograft can also be used in the manufacturing process for top layer 11. The patch can be manufactured entirely from allograft, completely replacing the usual animal fibers. Alternatively, the patch can be manufactured from a combination of allograft and animal collagen.

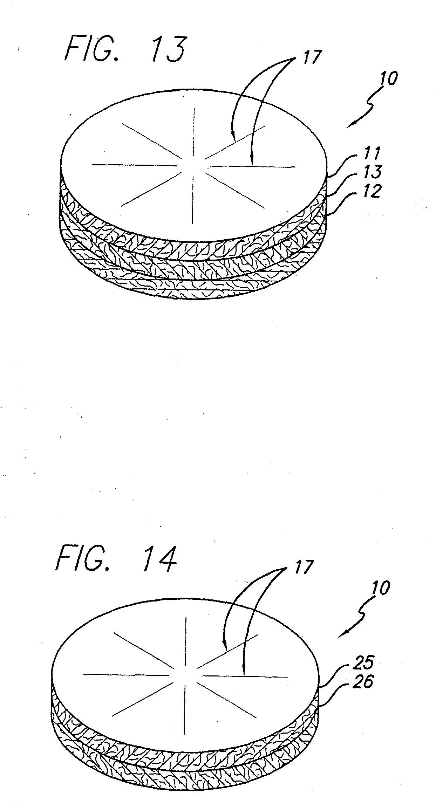

It will be apparent to those of skill in the art that the number and arrangement of layers 11, 12, and 13 can be varied without departing from the overall spirit and scope of the present invention. Figure 13 illustrates an alternative embodiment of the patch where the position of layers 12 and 13 are switched. In this embodiment, the barrier layer 13 is sandwiched between the layer of biological material 11 and the fabric 12. Figure 14 illustrates another alternative embodiment of the invention where the patch 10 has only two layers 25 and 26. In this embodiment the layer of fabric is omitted, and the patch consists of only a layer of biological material 25 and a layer of barrier material 26. Additional layers can also be added to the patch without departing from the scope of the invention. For example, a layer of medication may be added to the patch to decrease the chance of rejection by the body, to prevent infection, to reduce pain or to treat other complications that might arise during the surgical process.

The patch 10 is not limited to a particular size. The only real constraint on the size of the patch is that it must be able to fit within the space defined by the annulus fibrosus and end plates of an intervertebral disc. It has been found that the preferred thickness for the patch is approximately three millimeters. The instruments used in spinal surgery typically form holes in the annulus fibrosus that are less than seven millimeters in diameter. Therefore, the optimum size for a round patch has been found to be seven millimeters. The size of the patch may deviate from these dimensions if necessary. For example, it may be desired to increase the thickness of the top layer 11 by adding additional biological material. The total thickness is limited ony by the size available within the disc that is being repaired. The fact that the patch is relatively thin means that it can be easily trimmed to fit in the space required. A round patch can be trimmed to reduce it's over all diameter.

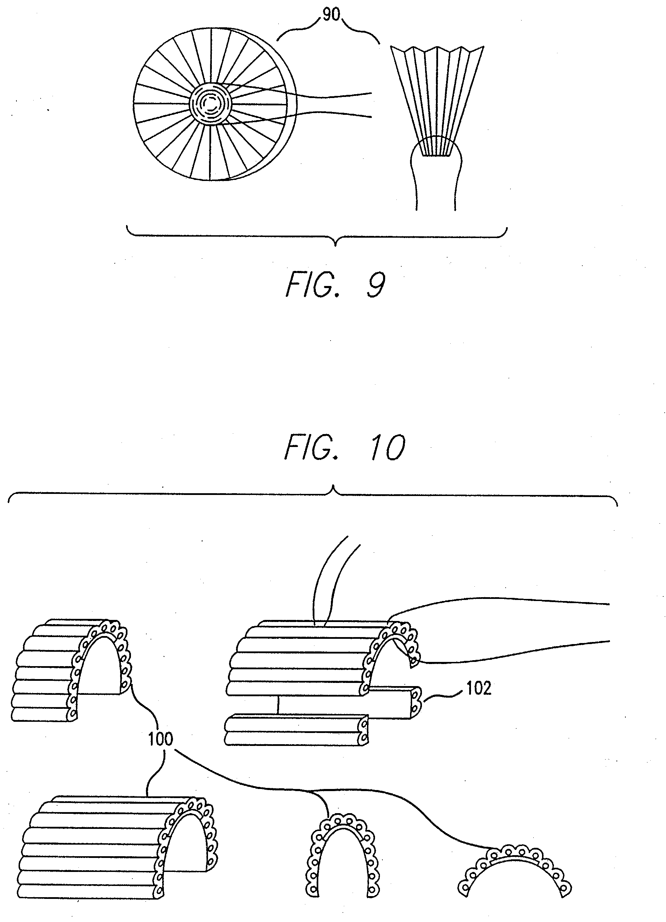

Figure 10 illustrates an alternative embodiment of the patch 100 which can be used to seal a multilateral annulotomy. A multilateral annulotomy 72 is shown in Figure 7b. The multilateral patch 100 is substantially rectangular in shape. The patch is flexible to allow for ease in placement through the delivery tube. The patch shown in Figure 10 can also be used to seal multiple fissures in the annulus. The size of the multilateral patch is approximately 5-10 millimeters on a side. The multilateral patch 100 is not limited to the exact shape and proportions shown in Figure 10. The shape shown has been found to be the most advantageous in sealing a multilateral annulotomy. It will be apparent to those of skill in the art that other shapes, proportions and arrangements can be used depending on the size, shape and location of the defect. As with a round patch, a multilateral patch 100 can be trimmed to size, if necessary, to reduce its length and width as depicted in element 120 in Figure 10.

Both the round 10 and multilateral 100 patches are typically folded during the process of inserting them into the disc. As a result, there is the potential for them to become bent and deformed. The patch may therefore by reinforced to resist bending after insertion. Reinforcement is accomplished by depositing semi-flexible small diameter filaments 17 on the top surface of the patch 10. The filaments are best illustrated in Figure 1, 13 and 14. The filaments are made of plastic or organic material. They are straight but flexible. They will regain their straight shape when bending moment is removed. For example, the collapsed round patch will open, and the folded multilateral patch will unfold once they are out of the delivery tube.

The re-enforcement members 17 in the multilateral patch are aligned in the longitudinal fashion to resist bending in its length. When unconstrained it unfolds into generally rectangular shape. The re-enforcement members 17 in the round patch is aligned in the radial orientation. When unconstrained, it opens to it's largest possible sized and resembles a circle. When introduced into the delivery tube, the round patch collapses and likens to a folded umbrella. (See element 110 in Figure 9). Once it is delivered deep to the annulus via the delivery tube it regain its open umbrella shape. The multilateral patch is introduced into the delivery tube in the folded configuration, but resumes its rectangular shape once it is out of the constrains of the delivery tube (See element 120 in Figure 12).

Referring again to Figure 8, the patch of the present invention is also provided with pull handles and tie down features. In its simple form one or more sutures 82 are placed in the patch. It has been found that the best results are achieved if the sutures are place around the sides of the multilateral patch, and at the center of the round patch. The suture handles 82 in the multilateral patch are used to pull the patch in a side-to-side direction in order to cover all the naturally occurred defects as well as the annulotomy hole. The suture 82 in the round patch is used to pull it firmly against the opening from within the disc. Once the patch is properly seated, it is tied downed to the annulus by using the suture. Tie-down of the patch prevents its gross movements.

Accordingly, a novel patch and patch material which can be used to repair defects in an intervertebral disc annulus has been described. The description of the present invention has been made with respect to specific arrangements and constructions of the preferred embodiment. It will be apparent to those skilled in the art that the foregoing description is for illustrative purposes only, and that various changes and modifications can be made to the present invention without departing from the overall spirit and scope of the present invention. The full extent of the present invention is defined and limited only by the following claims.

## Claims

1. A patch for repairing defects in a intervertebral disc annulus, said patch comprising:
biological material arranged in a substantially thin layer;
a fabric layer disposed underneath said biological material; and
means for connecting said biological material to said fabric layer.

2. The device of claim 1 wherein said patch is substantially circular in shape.

3. The device of claim 1 wherein said patch is substantially multilateral in shape.

4. The device of claim 1, wherein said biological material comprises human tissue.

5. The device of claim 4, wherein said human collagen is harvested from tissue belonging to a user of said patch.

6. The device of claim 4, wherein said human collagen is comprised of donor tissue.

7. The device of claim 1, wherein said means for connecting comprises an adhesive.

8. The device of claim 7 wherein said adhesive comprises biological glue.

9. The device of claim 8 wherein said means for connecting comprises a synthetic adhesive.

10. The device of claim 1 wherein said means for connecting comprises glue.

11. The device of claim 1 further comprising a layer of barrier material disposed underneath and coupled to said fabric layer, said barrier layer being resistant to liquids.

12. The device of claim 1 further comprising handles attached to said patch.

13. The device of claim 12 wherein said handles comprise sutures disposed through said patch.

14. A patch for repairing defects in a intervertebral disc annulus, said patch comprising:
a substantially thin layer of biological material;
a fabric layer disposed beneath said layer of animal collagen and coupled to said layer of animal collagen by a first adhesive means; and
a barrier layer of liquid resistant material disposed beneath said fabric layer, and coupled to said fabric layer by a second adhesive means.

15. The device of claim 14 wherein said patch is circular in shape.

16. The device of claim 14 wherein said fabric comprises cotton.

17. A patch for repairing defects in a intervertebral disc annulus, said patch comprising:
a substantially thin layer of biological material;
a barrier layer of liquid resistant material disposed beneath said fabric layer, and coupled to said fabric layer by a first adhesive means; and
a fabric layer disposed beneath said barrier layer and coupled to barrier layer by a second adhesive means; and

18. The device of claim 14 or claim 17 wherein said patch is circular in shape.

19. The device of claim 14 or claim 17 wherein said patch is rectangular in shape.

20. The device of claim 1 or claim 14 or claim 16 wherein said patch has a thickness of approximately three millimetres.

21. The device of claim 2 or claim 15 or claim 18 wherein said patch has a diameter of approximately seven millimetres.

22. The device of claim 1 or claim 14 or claim 17 wherein said fabric comprises cotton.

23. The device of claim 1 or claim 14 or claim 17 wherein said fabric comprises a synthetic polymer.

24. The device of claim 11 or claim 14 or claim 17 wherein said barrier layer is comprised of silicone.

25. The device of claim 11 or claim 14 or claim 17 wherein said barrier layer is comprised of plastic.

26. The device of claim 1 or claim 14 or claim 17 wherein said biological material comprises animal collagen.

27. The device of claim 1 or claim 14 or claim 17 wherein said biological material comprises human tissue.

28. The device of claim 14 or claim 17 wherein said first adhesive means comprises biological glue.

29. The device of claim 1 or claim 14 or claim 17 further comprising a plurality of flexible fibers disposed on a top surface of said patch.

30. The device of claim 29 wherein said patch is circular in shape and said flexible fibers are oriented in a radial direction.

31. The device of claim 14 or claim 17 further comprising at least one suture disposed through said patch, wherein said suture functions as a handle after said patch has been placed in a desired location within said disc.

32. A patch for repairing defects in a intervertebral disc annulus, said patch comprising:
a substantially thin layer of animal collagen arranged in a substantially circular shape;
a barrier layer of liquid resistant material disposed beneath said layer of animal collagen, and coupled to said layer of animal collagen by biological glue;
a plurality of flexible fibers disposed on a top surface of said patch and oriented in a radial direction; and
at least one suture disposed through said patch, said suture acting as a handle.

33. A patch for repairing defects in a intervertebral disc annulus, said patch comprising:
a substantially thin layer of animal collagen arranged in a substantially rectangular shape;
a barrier layer of liquid resistant material disposed beneath said layer of animal collagen, and coupled to said layer of animal collagen by biological glue;
a plurality of flexible fibers disposed on a top surface of said patch and oriented in a radial direction; and
at least one suture disposed through said patch, said suture acting as handle.

34. A patch for repairing defects in a intervertebral disc annulus, said patch comprising:
a substantially thin layer of animal collagen arranged in a substantially circular shape;
a fabric layer disposed beneath said layer of animal collagen and coupled to said layer of animal collagen by biological glue;
a barrier layer of liquid resistant material disposed beneath said fabric layer, and coupled to said fabric layer by an adhesive;
a plurality of flexible fibers disposed on a top surface of said patch and oriented in a radial direction; and
at least one suture disposed through said patch, said suture acting as a handle.

35. A patch for repairing defects in a intervertebral disc annulus, said patch comprising:
a substantially thin layer of animal collagen arranged in a substantially circular shape;
a barrier layer of liquid resistant material disposed beneath said layer of animal collagen, and coupled to said layer of animal collagen by biological glue;
a fabric layer disposed beneath said barrier layer and coupled to said barrier layer by an adhesive;
a plurality of flexible fibers disposed on a top surface of said patch and oriented in a radial direction; and
at least one suture disposed through said patch, said suture acting as a handle.

36. A patch for repairing defects in a intervertebral disc annulus, said patch comprising:
a substantially thin layer of animal collagen arranged in a substantially rectangular shape;
a fabric layer disposed beneath said layer of animal collagen and coupled to said layer of animal collagen by biological glue;
a barrier layer of liquid resistant material disposed beneath said fabric layer, and coupled to said fabric by an adhesive;
a plurality of flexible fibers disposed on a top surface of said patch; and
at least one suture disposed through said patch, said suture acting as a handle.

37. A patch for repairing defects in a intervertebral disc annulus, said patch comprising:
a substantially thin layer of animal collagen arranged in a substantially rectangular shape;
a barrier layout of liquid resistant material disposed beneath said layer of animal collagen, and coupled to said layer of animal collagen by biological glue;
a fabric layer disposed beneath said barrier layer and coupled to said barrier layer by an adhesive;
a plurality of flexible fibers disposed on a top surface of said patch and;
at least one suture disposed through said patch, said suture acting as a handle.
